Europäisches Patentamt

European Patent Office

Office européen des brevets

(19)

(11) Publication number: **0 250 129**
**A2**

(12) **EUROPEAN PATENT APPLICATION**

(21) Application number: **87304917.5**

(22) Date of filing: **03.06.87**

(51) Int. Cl.⁴: **A61D 7/00 , B65D 47/34**

(30) Priority: **12.06.86 US 873341**

(43) Date of publication of application:
**23.12.87 Bulletin 87/52**

(84) Designated Contracting States:
**AT BE CH DE ES FR GB IT LI LU NL SE**

(71) Applicant: **A.H. ROBINS COMPANY,**
**INCORPORATED**
**1405 Cummings Drive PO Box 26609**
**Richmond Virginia 23261-6609(US)**

(72) Inventor: **Blutenthal, John Edward**
**2141 Oakengate Lane Midlothian**
**Virginia 23113(US)**

(74) Representative: **Sheard, Andrew Gregory et al**
**Kilburn & Strode 30, John Street**
**London WC1N 2DD(GB)**

(54) **Lip medication applicator.**

(57) An applicator for administering medicated compositions to the lip area of a living animal has a container (1) for storing the medicated composition with a tip area (2) with an angled delivery surface (3). An orifice (6) connects the angled delivery surface with the interior of the tip area and container so that upon pressure being administered to the outer walls of the container (7) the medicated composition will pass from the container through the orifice (6) onto the angled delivery surface for administration to the lip area of the animal. The container (1) may have threads (4) for securing a cap over the tip area (2) of the container.

*FIG. 2*

EP 0 250 129 A2

## LIP MEDICATION APPLICATOR

This invention relates to a lip medication applicator and more particularly relates to a lip applicator with a tip area having an angled delivery surface for better and more efficient application of medicated compositions to the lips.

Previously, commercially available applicators for delivering medication to the lips had a rounded surface from which the medication was released upon activation. Administration of such medication to the lips from this rounded surface made even distribution of the medication on the entire lip surface (including narrowed angles and curves) difficult and the medication was usually administered in an uneven and uncontrolled manner.

It is an object of this invention to provide an applicator having an angled delivery surface so that medication may be administered to the lips in a smooth and even manner.

It is a further object of the invention to provide an applicator having an angled delivery surface so that medication may be administered to the lips in a controlled manner.

It is another object of this invention to provide an applicator having an angled delivery surface so that the angle of the surface will match the contour of the lip surface in order to provide a controlled and even distribution of medication to the lip surface.

The applicator device of this invention comprises a container with a tip area having an angled delivery surface with an orifice present through this surface to connect the interior of the container to the angled delivery surface so that the desired medication may be released from the interior of the container onto the angled delivery surface for administration of the said medication to the lip surface.

According to the present invention there is provided an applicator useful in administering medicated compositions to the lip area of a living animal which comprises a container for storing the medicated composition and having a tip area with an angled delivery surface and an orifice connecting the angled delivery surface with the interior of the tip area and container so that upon pressure being administered to the outer walls of the container the medicated composition will pass from the container through the orifice onto the angled delivery surface for administration to the lip area of the animal.

For a better understanding of the invention, and to show how it may be put into effect, reference will now be made by way of example to the accompaying drawings, in which:

Figure 1 shows the applicator having the angled tip delivery surface.

Figure 2 shows an enlargement of the angled tip delivery surface.

Figure 3 shows a perspective enlargement of the angled tip delivery surface.

An applicator of this invention as shown in the drawings comprises a container 1 with a tip area 2 having an angled delivery surface 3. The container 1 contains the medicated composition for administration which is forced into the tip area 2 of the container by pressure on the container sides 7 and is then released onto the angled delivery surface 3 of the tip through orifice 6 which connects the container and tip interior with the angled delivery surface. Optional threads 4 on the container 1 may be used to secure a cap (not shown) over the tip area 2 of the container when not in use. Preferably the applicator will be provided with a cap. Preferably, the cap will be equipped with a protruding member so that when in place over the tip area of the container, the protruding member will fit into orifice 6 to retain the medicated composition within the container and prevent leakage of the medicated composition from the container.

The container may be conveniently formed by any conventional moulding procedure. The container is then filled with the medicated composition through the opening 5. The container is then sealed at opening 5. This may be conveniently done by a conventional heat sealing technique.

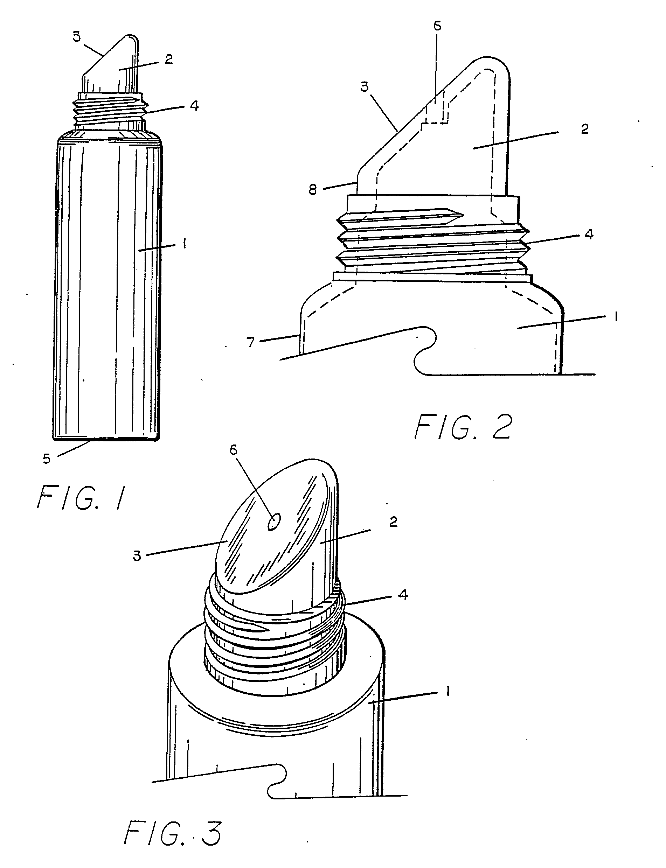

The container is normally made of a plastics material, such as polyethylene, which may be moulded into the desired shape as shown in Figure 1. The walls of the container below the tip area are thinly formed as shown at 7 in Figure 2, as it is only necessary that the outer walls 7 of the container be thin so that they will depress upon pressure to force the medicated composition into the tip area 2 and onto the angled delivery surface 3 through orifice 6 which connects angled delivery surface 3 with the interior of the tip area 2 and container 1. Upon the release of the pressure on the outer walls of the container 1, the walls of the container will revert to their original position.

The angled delivery surface 3 of the tip area 2 should be formed at an angle of about 30°-60° from the vertical line 8 of the tip area as shown in Figure 2. Preferably, the angled delivery surface 3 of the tip area 2 is formed at an angle of 40° to 50° from the vertical line (8) of the tip area (2). Preferably, the angled delivery surface 3 of the tip area 2 is formed at an angle of 43° to 47° from the vertical line (8) of the tip area (2). Preferably the angled delivery surface of the tip area will be formed at an angle of about 45° from the vertical of the tip area of the container. This angle has been found to be preferable as its contour best mirrors the lip to which the medication is to be applied.

The medicated composition which may be administered using the applicator of this invention may be any (eg any conventional) composition useful in treating the lip area and is conveniently administered in the form of a cream, balm, jelly, ointment, salve, or the like. The administration of solid compositions would not be contemplated using the applicators of this invention. The following compositions are illustrative of medicated compositions which may be applied to the lip area using the applicator of this invention.

| Composition A | | Composition B | |
|---|---|---|---|
| Aloe Vera Oil | 0.5% | Aloe Vera Oil | 0.5% |
| Lanolin, anhydrous | 0.5% | Lanolin, anhydrous | 0.5% |
| White petrolatum | 99% | Sun screen | 10% |
| | | White petrolatum | 89% |

Percentages given are by weight of the total weight of the composition.

**Claims**

1. An applicator useful in administering medicated compositions to the lip area of a living animal which comprises a container (1) for storing the medicated composition and having a tip area (2) with an angled delivery surface (3) and an orifice (6) connecting the angled delivery surface with the interior of the tip area and container so that upon pressure being administered to the outer walls of the container (7) the medicated composition will pass from the container through the orifice (6) onto the angled delivery surface for administration to the lip area of the animal.

2. An applicator according to claim 1 in which the container (1) has threads (4) thereon securing a cap over the tip area (2) of the container (1).

3. An applicator according to any of the preceding claims in which the applicator has a cap with a protruding member so that when the protruding member is secured over the tip area (2) of the container (1), the protruding member will fit into the orifice (6) of the tip area (2).

4. An applicator according to any of the preceding claims in which the angled delivery surface (3) of the tip area is formed at an angle of 30° to 60° from the vertical line (8) of the tip area (2).

5. An applicator according to any of the preceding claims in which the angled delivery surface (3) of the tip area (2) is formed at an angle of 40° to 50° from the vertical line (8) of the tip area (2).

6. An applicator according to any of the preceding claims in which the angled delivery surface (3) of the tip area (2) is formed at an angle of 43° to 47° from the vertical line (8) of the tip area (2).

7. An applicator according to any of the preceding claims in which the angled delivery surface (3) of the tip area (2) is formed at about a 45° angle with the vertical line (8) of the tip area (2).

8. An applicator according to any of the preceding claims in which the container (1) is formed of a plastics material.

9. An applicator according to any of the preceding claims in which the container (1) is formed by a moulding procedure.

10. An applicator according to claim 8 or claim 9 in which the plastics material is polyethylene.

3

Neu eingereicht / Newly filed
Nouvellement déposé

FIG. 1

FIG. 2

FIG. 3